**Europäisches Patentamt**

**European Patent Office**

**Office européen des brevets**

(11) Numéro de publication: **0 296 965 B1**

## FASCICULE DE BREVET EUROPEEN

(45) Date de publication de fascicule du brevet: **20.10.93**

(51) Int. Cl.5: **C12P 19/06**, C12N 1/20

(21) Numéro de dépôt: **88401562.9**

(22) Date de dépôt: **22.06.88**

(54) **Procédé de fermentation pour l'obtention d'un polysaccharide de type xanthane.**

(30) Priorité: **22.06.87 FR 8708727**

(43) Date de publication de la demande:
**28.12.88 Bulletin 88/52**

(45) Mention de la délivrance du brevet:
**20.10.93 Bulletin 93/42**

(84) Etats contractants désignés:
**AT BE CH DE ES GB GR IT LI LU NL SE**

(56) Documents cités:
**US-A- 3 427 226**

**PATENT ABSTRACTS OF JAPAN, vol. 10, no. 49 (C-330)[2106], 26 février 1986, page 147 C 330**

(73) Titulaire: **ELF SANOFI**
**32-34, rue Marbeuf**
**F-75008 Paris(FR)**

(72) Inventeur: **Eyssautier, Bruno**
**8, Cité Auby**
**F-50500 Carentan(FR)**

(74) Mandataire: **Gillard, Marie-Louise et al**
**Cabinet Beau de Loménie**
**158, rue de l'Université**
**F-75340 Paris Cédex 07 (FR)**

Rank Xerox (UK) Business Services
(3. 10 / 3.6 / 3.3. 1)

**EP 0 296 965 B1**

## Description

La présente invention concerne un procédé de fermentation d'hydrates de carbone pour l'obtention d'un polysaccharide de type xanthane au moyen de microorganismes du type Xanthomonas, et le polysaccharide obtenu par ce procédé.

On sait que la culture aérobie de bactéries du type Xanthomonas dans des milieux aqueux de pH compris entre 5,5 et 9 contenant au moins une source de carbone, une source d'azote, des ions phosphates et des oligoéléments permet d'obtenir une composition de polysaccharide du type xanthane. La source de carbone est généralement constituée par des hydrates de carbone tandis qu'on a décrit l'utilisation comme source d'azote de dresches sèches de distillerie dans US 3.000.790, de peptones dans TAPPI 5 p. 442-445 de M.O. BAGBY, I.A. WOLFF et M.C. CADMUS, d'extraits de levure dans Biotechnol. Bioeng (1971) 13 p. 381 de R.A. MORAINE et P. ROGOVIN, de liqueurs de macération dans US 3.335.447 ou de farines de soja dans US 3.271.267 ; des farines de céréales comme celles des sorgho apportent à la fois la source de carbone et celle d'azote comme décrit dans US 3.271.267.

Les produits couramment utilisés comme source d'azote introduisent dans le milieu des impuretés insolubles qui se retrouvent dans le polysaccharide isolé en fin de fermentation ce qui implique, pour certaines des applications du polysaccharide, de traiter le produit obtenu par divers procédés, tels que par exemple filtration ou floculation pour éliminer ces impuretés insolubles qui conduisent à la formation de troubles dans les solutions aqueuses de polysaccharide.

Par ailleurs, le xanthane est utilisé, pour son pouvoir épaississant pratiquement indépendant de la température et du pH, dans la récupération assistée du pétrole, comme additif alimentaire dans des formulations pharmaceutiques ou cosmétiques, ou dans la préparation de textiles ou d'explosifs et il est particulièrement important d'avoir une qualité de xanthane qui naturellement a un fort pouvoir épaississant, c'est-à-dire dont les solutions aqueuses présentent une forte viscosité.

La présente invention concerne un procédé de fermentation qui, grâce à l'utilisation d'une source d'azote convenablement choisie, permet d'obtenir un polysaccharide de type xanthane à fort pouvoir épaississant, contenant peu d'insolubles, et dont les solutions aqueuses sont limpides et très peu colorées, ce qui est avantageux pour de nombreuses applications.

Selon l'invention, on utilise comme source d'azote soluble en milieu aqueux, de la gélatine et plus particulièrement une gélatine de faible masse moléculaire. On sait qu'il existe deux types de gélatine : la gélatine de type A qui est obtenue par hydrolyse acide, par exemple par l'acide sulfurique, du collagène présent dans les peaux ou dans les os de porcs et de bovins, et la gélatine de type B qui est obtenue par hydrolyse basique, notamment par $Ca(OH)_2$ , du collagène. Les gélatines courantes, de masse moléculaire supérieure ou égale à 20 000 sont des sources d'azote connues, mais qui ne permettent pas d'obtenir les résultats avantageux de l'invention. Il est souhaitable d'utiliser des gélatines de masse moléculaire moyenne inférieure à 5 000, pratiquement sans force en gelée ; les gélatines hydrolysées de masse moléculaire comprise entre 1 500 et 2 500 sont particulièrement préférées.

On peut introduire dans le milieu de culture une gélatine préalablement hydrolysée soit par chauffage en milieu acide aqueux à un pH <4 et à une température supérieure à 100°C, ou encore par action d'une protéase comme décrit dans le brevet FR. 1.501.821.

Mais, le gélatine peut aussi être hydrolysée, au moins partiellement, au cours de la stérilisation du milieu de culture, avant l'introduction des microorganismes si le pH, la température et la durée du chauffage, sont convenablement choisis ; dans ce cas on préfère introduire au départ des gélatines dont la masse moléculaire, avant la stérilisation hydrolysante, n'est pas supérieure à 10 000.

Lorsque la stérilisation est effectuée à un pH sensiblement neutre ou à pH faiblement acide, pendant des temps très courts, inférieurs à 5 minutes, la gélatine mise en oeuvre devra avoir une masse moléculaire inférieure à 5 000 et de préférence inférieure à 3 000.

La quantité de gélatine introduite dans le milieu de fermentation est choisie pour obtenir une teneur en azote total de 0,1 g/l à 1 g/l.

Le milieu peut aussi contenir de façon classique de 5 g/l à 50 g/l d'hydrates de carbone, tels que le glucose, les amidons natifs ou hydrolysés, le saccharose, le lévulose, le fructose, le maltose, les mélasses de betterave ou de canne à sucre, ainsi que de 0,10 à 20 g/l, et de préférence 0,5 à 5 g/l de phosphates, tel que $K_2HPO_4$, et un ou plusieurs oligo-éléments dont du magnésium à la concentration de 0,025 à 1 g/l de magnésium sous forme d'un sel de magnésium soluble, tel que le sulfate, l'acétate, le chlorure et le nitrate de magnésium.

Les bactéries qui permettent d'obtenir un xanthane par fermentation dans ces milieux sont en général du genre Xanthomonas, elles peuvent appartenir à différentes espèces comme Xanthomonas begoniae, Xanthomonas incanae, Xanthomonas vesicatoria, Xanthomonas campestris et Xanthomonas phaesoli et

2

autres espèces bien connues de l'homme du métier ; on utilise généralement des souches de <u>Xanthomonas</u> <u>campestris</u>.

La fermentation est effectuée à un pH qui peut être compris entre 5,5 et 9, et de préférence entre 6,5 et 8, à une température comprise entre 25°C et 35°C et de préférence entre 27°C et 32°C. Le milieu est agité et aéré de façon classique ; dans ces conditions la durée de la fermentation est de 1 à 6 jours.

De façon classique, le procédé mis en oeuvre pour obtenir le polysaccharide selon l'invention comporte plusieurs étapes :

1/ Préparation d'un inoculum à partir d'une souche de <u>Xanthomonas</u> qui pouvait être congelée ou lyophilisée ;

2/ éventuellement, croissance des microorganismes dans un milieu de préculture ;

3/ production du polysaccharide dans le milieu de fermentation, qui a été inoculé avec les milieux obtenus à l'étape 1 ou 2 ;

4/ et isolement du polysaccharide.

L'isolement du polysaccharide se fait en général par précipitation, en introduisant dans le milieu de fermentation un solvant dans lequel le polysaccharide est insoluble. Parmi les solvants généralement utilisés, on peut citer les alcools inférieurs, tels que le méthanol, l'éthanol, l'isopropanol et le butanol, ou l'acétone ; on préfère l'isopropanol. On peut avant l'étape de précipitation, chauffer le milieu réactionnel à une température comprise entre 80°C et 130°C, pendant quelques minutes pour détruire les bactéries.

On peut aussi traiter avant, ou après la stérilisation, le milieu par un dialdéhyde aliphatique, tel que le glyoxal, moyen connu pour améliorer la dispersibilité aqueuse des polysaccharides, le xanthane est ensuite précipité dans le milieu, isolé par filtration, lavé avec le solvant précipitant puis séché et broyé si nécessaire. On utilise dans ce cas de 0,1 à 1 % en poids de glyoxal par rapport au poids de xanthane sec et en général environ 2 %. Le xanthane peut aussi être traité par un dialdéhyde, après son isolement, si l'opération n'a pas été effectuée dans le milieu de fermentation. Un procédé avantageux consiste à humidifier la poudre de xanthane par une solution de glyoxal dans l'isopropanol aqueux, préparée par mélange de 5 à 10 ml de solution aqueuse de glyoxal à 30 % avec 150 ml d'isopropanol ; après 20 à 40 minutes de contact, les solvants sont éliminés par chauffage et le polysaccharide sec peut alors etre stocké pour être ultérieurement utilisé dans ses applications classiques.

Pour améliorer les conditions de fermentation, et notamment diminuer la viscosité du milieu, on peut aussi effectuer la fermentation sur gélatine en émulsion, en présence d'une huile et d'un tensioactif selon le procédé décrit dans EP-A-58364 mettant en oeuvre une émulsion eau-dans-l'huile comme milieu de fermentation, ou le procédé décrit dans EP-A-187092 mettant en oeuvre une émulsion huile-dans-l'eau comme milieu de fermentation.

Un autre objet de l'invention est la composition de polysaccharide du type xanthane obtenue par le procédé de fermentation selon l'invention.

Cette composition de polysaccharide est constituée, comme tous les produits de ce type, d'unités saccharidiques de type mannose, glucose, et acide glucuronique salifié ; certains des groupes hydroxyles sont estérifiés par l'acide acétique et de l'acide pyruvique est lié, par une fonction acétal, à certaines unités mannose. La masse moléculaire de la composition de polysaccharide selon l'invention est supérieure à 2 000 000.

Ses solutions aqueuses ont une viscosité particulièrement élevée, supérieure à celle des solutions de xanthane produit par fermentation dans un milieu contenant de la farine de soja ou des liqueurs de macération du maïs, comme source d'azote.

Ainsi une solution aqueuse contenant 1 % en poids de KCl et 0,2 % en poids du polysaccharide de l'invention a une viscosité de 200 mPa.s, au moins, mesurée au viscosimètre Brookfield à 24°C, alors que, dans les mêmes conditions, celle d'une solution aqueuse du polysaccharide obtenu par fermentation sur farine de soja est de 160 mPa.s environ. Les solutions aqueuses du polysaccharide selon l'invention ont une faible densité optique ; par exemple, les solutions contenant 0,2 % en poids de polysaccharide ont une densité optique, mesurée à 620 nm, inférieure ou égale à 0,2.

Dans ce qui suit, on décrit un exemple de mise en oeuvre de l'invention.

**EXEMPLE**

a) Préparation de l'inoculum :

On ensemence avec 3 ml de bactéries décongelées de la souche de <u>Xanthomonas campestris</u> déposée sous le n° NRRL.B 1459, un milieu MY de culture de revitalisation constitué de :

| Glucose | 10,0 g |
|---|---|
| Pentone | 0,5 g |
| Extrait de levure | 0,3 g |
| Extrait de malt | 3,0 g |
| Eau distillée | 1000 g |

préalablement stérilisé par chauffage durant 20 min à 120°C.

Après ensemencement, le milieu est mis à incuber sous agitation à 30°C pendant 12 à 18 heures.

b) Préculture :

La croissance des microorganismes de l'inoculum a lieu ensuite dans un fermenteur de préculture contenant 10 l du milieu constitué de :

| Glucose | 150,0 g |
|---|---|
| Gélatine | 20,0 g |
| $K_2HPO_4$ | 8,0 g |
| Mg SO$_4$ | 1,8 g |
| Antimousse | 0 à 3,0 g |
| Eau | qsp 10 l. |

Le glucose est préalablement stérilisé par chauffage à pH 4, tandis que les autres constituants du milieu sont stérilisés en mélange, par exemple par chauffage à pH 4,5 et à 120°C pendant 30 minutes.

La préculture est poursuivie pendant 20 heures avant de servir à ensemencer le milieu de fermentation.

c) Fermentation et production du polysaccharide:

Le milieu de fermentation pour un fermenteur de volume utile de 100 l est constitué de :

| Glucose | 3 kg |
|---|---|
| Gélatine | 200 g |
| $K_2HPO_4$ | 80 g |
| MgSO$_4$ | 18 g |
| Antimousse | 0 à 30 g |
| Eau | qsp 100 l |

Le glucose est stérilisé seul à pH 4 comme précédemment, les autres constituants sont stérilisés à 120°C pendant 30 minutes, à différents pH, ce qui conduit à des gélatines de masses moléculaires différentes.

Avant l'ensemencement avec l'inoculum préparé dans l'étape b, le pH du milieu est ajusté à pH 7, et maintenu autour de cette valeur pendant toute la durée de la fermentation.

De façon classique, le milieu est agité et aéré pendant toute la fermentation.

On indique dans le tableau I, les résultats obtenus en utilisant deux gélatines de type A de masse moléculaire 5 000, sans force en gelée et de point isoélectrique 6,3 ou 6,6, hydrolysées lors de la stérilisation à différents pH.

La gélatine 1 est commercialisée par la Cie Rousselot sous la référence ASF, et la gélatine 2 sous la référence HP50.

A titre de comparaison, on a aussi effectué une fermentation en utilisant de la farine de soja comme source d'azote.

Le xanthane a été précipité à la fin de la fermentation par introduction de 200 l d'isopropanol dans le milieu ; il a été ensuite séparé par filtration et séché par chauffage sous courant d'air.

**TABLEAU 1**

| Source d'azote | pH de stérilisation | Masse moléculaire | Durée de la fermentation | Poids de xanthane g/kg milieu | Productivité g/L/h | Viscosité de la solution (a) mPa.s | Densité optique (b) |
|---|---|---|---|---|---|---|---|
| Farine de soja | 7 | | 47 h | 19,2 | 0,40 | 160 | 0,35 |
| Gélatine 1 | 7 | 5 000 | 70 h | 5 | 0,071 | – | – |
| | 4 | 4 000 | 63 h | 20 | 0,32 | 230 | 0,2 |
| | 2 | 2 000 | 55 h | 22,6 | 0,41 | 205 | 0,194 |
| Gélatine 2 | 4 | 2 500 | 54 h | 22,5 | 0,42 | 220 | 0,168 |
| | 2 | 2 000 | 53 h | 22,7 | 0,43 | 225 | 0,174 |

a) concentration 0,2 % en poids dans solution aqueuse contenant 1 % en poids de KCl ; mesurée au viscosimètre Brookfield à T =

b) en solution aqueuse à 0,2% en poids et mesurée à une longeur d'onde de 620 nm.

**Revendications**

1. Procédé de fermentation d'hydrates de carbone par des bactéries du genre Xanthomonas pour la production d'un polysaccharide du type xanthane, caractérisé en ce que la source d'azote est constituée d'une gélatine hydrolysée de masse moléculaire inférieure à 5 000.

**2.** Procédé de fermentation selon la revendication 1, caractérisé en ce que la gélatine hydrolysée a une masse moléculaire comprise entre 1 500 et 2 500.

**3.** Procédé selon l'une des revendications 1 ou 2, caractérisé en ce que les bactéries appartiennent à l'espèce Xanthomonas campestris.

**4.** Procédé de fermentation selon l'une quelconque des revendications 1 à 3, caractérisé en ce que le milieu comprend de 5g/l à 50g/l d'hydrates de carbone, de 0,5 g/l à 5 g/l de $K_2HPO_4$, de 0,025 g/l à 1 g/l de magnésium et une quantité suffisante de gélatine dépolymérisée pour apporter de 0,1 g/l à 1 g/l d'azote total.

**5.** Procédé de fermentation selon l'une quelconque des revendications 1 à 4, caractérisé en ce que l'hydrolyse de la gélatine a lieu au moins partiellement, au cours de la stérilisation du milieu de fermentation.

**6.** Procédé d'obtention d'un polysaccharide du type xanthane qui consiste à :
a) préparer un inoculum de bactéries du genre Xanthomonas ;
b) fermenter des hydrates de carbone par lesdites bactéries par le procédé selon l'une des revendications 1 à 5 ;
c) et à isoler le polysaccharide obtenu.

**7.** Composition de polysaccharide du type xanthane susceptible d'être préparée par le procédé selon la revendication 6, dont les solutions aqueuses à 0,2 % en poids en présence de KCl ont une viscosité supérieure à 200 mPa.s et dont les solutions aqueuses à 0,2 % en poids ont une densité optique mesurée à 620 nm inférieure ou égale à 0,2.

**Claims**

**1.** Process far the fermentation of carbohydrates with bacteria of the genus Xanthomonas for the production of a polysaccharide of the xanthan type, characterized in that the source of nitrogen consists of a hydrolyzed gelatin with a molecular weight of less than 5000.

**2.** Fermentation process according to claim 1, characterized in that the hydrolyzed gelatin has a molecular weight included between 1500 and 2500.

**3.** Process according to one of claims 1 or 2, characterized in that the bacteria belong to the species Xanthomonas campestris.

**4.** Fermentation process according to any one of claims 1 to 3, characterized in that the medium comprises from 5 g/l to 50 g/l of carbohydrates, from 0.5 g/l to 5 g/l of $K_2HPO_4$, from 0.025 g/l to 1 g/l of magnesium and a sufficient amount of depolymerized gelatin to provide from 0.1 g/l to 1 g/l of total nitrogen.

**5.** Fermentation process according to any one of claims 1 to 4, characterized in that the gelatin is at least partially hydrolyzed during sterilization of the fermentation medium.

**6.** Process for the preparation of a polysaccharide of the xanthan type which consists in :
a) preparing an inoculum of bacteria of the genus Xanthomonas;
b) fermenting carbohydrates with the said bacteria by the process according to one of claims 1 to 5;
c) isolating the polysaccharide obained.

**7.** Polysaccharide composition of the xanthan type, obtainable by the process according to claim 6, whose viscosity in aqueous solutions containing 0.2% by weight in the presence of KCl, is greater than 200 mPa.s, and whose optical density in aqueous solutions containing 0.2% by weight, measured at 620 nm, is less than or equal to 0.2.

**Patentansprüche**

1. Fermentationsverfahren von Kohlehydraten mittels Bakterien der Gattung Xanthomonas zur Herstellung eines Polysaccharids vom Xanthan-Typ, dadurch gekennzeichnet, daß die Stickstoffquelle aus einer hydrolysieren Gelatine mit einem Molgewicht von weniger als 5000 besteht.

2. Fermentationsverfahren nach Anspruch 1, dadurch gekennzeichnet, daß die hydrolysierte Gelatine ein Molgewicht von 1500 bis 2500 aufweist.

3. Verfahren nach einem der Ansprüche 1 oder 2, dadurch gekennzeichnet, daß die Bakterien der Art Xanthomonas campestris angehören.

4. Fermentationsverfahren nach einem der Ansprüche 1 bis 3, dadurch gekennzeichnet, daß das Medium pro 5 bis 50 g/l Kohlehydrate, 0,5 bis 5 g/l $K_2HPO_4$, 0,025 bis 1 g/l Magnesium und eine ausreichende Menge an depolymerisierter Gelatine zum Vorsehen von 0,1 bis 1 g/1 Gesamtstickstoff enthält.

5. Fermentationsverfahren nach einem der Ansprüche 1 bis 4, dadurch gekennzeichnet, daß die Hydrolyse der Gelatine während der Sterilisation des Fermentationsmediums zumindest teilweise erfolgt ist.

6. Verfahren zum Herstellen eines Polysaccharids vom Xanthan-Typ, das darin besteht, daß
   a) ein Inokulum von Bakterien der Gattung Xanthomonas hergestellt wird,
   b) Kohlehydrate durch diese Bakterien nach dem Verfahren nach einem der Ansprüche 1 bis 5 fermentiert werden und
   c) das erhaltene Polysaccharid isoliert wird.

7. Polysaccharidzusammensetzung vom Xanthan-Typ, herstellbar durch das Verfahren nach Anspruch 6, deren 2 gew.%ige Lösungen in Anwesenheit von KCl eine Viskosität von höher als 200 mPa.s haben und deren 0,2 gew.%ige wässerige Lösungen eine optische Dichte, gemessen bei 620 nm, von niedriger oder gleich 0,2 aufweisen.